# EUROPEAN PATENT SPECIFICATION

(11) **EP 3 965 858 B1**
(45) Date of publication and mention of the grant of the patent: **03.07.2024**
(21) Application number: 19725432.9
(22) Date of filing: 09.05.2019
(51) Int. Cl.: A61M 16/00, A61M 16/10, A61M 16/12

(54) **FLUSHING OF VENTILATOR BREATHING SYSTEM**
SPÜLUNG EINES BEATMUNGSSYSTEMS
RINÇAGE D'UN SYSTÈME RESPIRATOIRE À VENTILATEUR

(43) Date of publication of application: 16.03.2022
(73) Proprietor: Maquet Critical Care AB, 171 06 Solna (SE)
(72) Inventor: LARSSON, Åke, 175 64 Järfälla (SE); MAKSUTI, Elira, 129 42 Hägersten (SE)
(74) Representative: Zacco Sweden AB
(86) International application number: PCT/SE2019/050414
(87) International publication number: WO 2020/226543

(56) References cited:
- WO-A1-2016/209129
- US-A1- 2012 090 611

## Description

### TECHNICAL FIELD

The present disclosure relates to a ventilator, an unclaimed method and a computer program for delivering oxygen-containing breathing gas to a patient via a ventilator breathing system (VBS) connecting the ventilator and the patient.

### BACKGROUND

Most modern ventilators offer a function, herein referred to as "oxygen boost", which allows the operator of the ventilator to temporarily increase the oxygen concentration of the breathing gas delivered to the patient. Typically, when the operator activates the oxygen boost function, the ventilator automatically increases the fraction of delivered oxygen by a fixed amount for a fixed period of time, e.g. by having the ventilator delivering 100% oxygen instead of air for one minute of time.

However, it is difficult in clinical practice to foresee the effect of such an oxygen boost since it is difficult to know how the increase in ventilator-delivered fraction of oxygen affects the oxygen concentration in the breathing gas actually reaching the patient, at least unless monitoring said oxygen concentration by means of an oxygen sensor disposed in the proximity of the patient, e.g. in or near the patient end adapter of the VBS connecting the patient and the ventilator.

Often, there is a considerable delay in oxygen concentration increase in the breathing gas reaching the patient. In fact, during ventilation treatment of neo-patients where tidal volumes are small compared to the volumes of hoses and additional equipment such as humidifiers, there may be a delay in delivery of increased oxygen concentration to the patient of nearly one minute, meaning that there may be no or nearly no increase in oxygen concentration in the breathing gas delivered to the patient even one minute after activation of the oxygen boost function.

Furthermore, clinicians are often mislead by the erroneous conception that activation of the oxygen boost function causes a momentary increase in oxygen concentration to a preset level (oxygen boost level), and that breathing gas with an oxygen concentration corresponding to said oxygen boost level is delivered to the patient from the point in time of activation of the oxygen boost function and for a set period of time during which the oxygen boost function is activated (oxygen boost duration).

This misconception may give rise to situations in which activation of the oxygen boost function does not have the desired effect on the ventilated patient. In some cases, for example in situations in which the oxygen boost function is activated in order to temporarily increase the patient's blood oxygen levels before disconnecting the patient from the ventilator, the unawareness of this delay may put the patient in a state of hypoxia.

This problem may be mitigated to some extent by determining and indicting to the ventilator operator the delay in delivery of the oxygen-enriched gas to the patient, as suggested in WO 2016/209129A1.

Although visualizing the delay in delivery of the oxygen-enriched gas to the patient may improve understanding of the oxygen boost functionality of the ventilator, the underlying problem of the patient not receiving oxygen-enriched gas in due time after a change in oxygen concentration still remains.

International patent publication WO 2016/209129 discloses a method of providing an oxygen boost to the patient and ensuring that the boost actually reaches the patient by compensating for the delay in delivery of the changed gas composition to the patient. According to this disclosure, a delay time is calculated on the basis of i.a. the volume of the inspiratory line, to determine when the boost reaches the patient. The delay time is not affected.

US 2012/090611 discloses a method of reducing the delay in delivery, or washout time, in a ventilator when changing the gas composition, to reduce the time until the new composition reaches the patient. This is achieved by momentarily opening the inspiration and expiration valves simultaneously during expiration while providing a bias flow in the inspiration line. This method allows for the washout procedure to be performed only during a very short time and preferably during expiration, so that the patient will not be affected by the pressure changes caused by opening the inspiration and expiration valves.

### SUMMARY

It is an object of the present disclosure to present means for improved patient safety during mechanical ventilation.

It is another object of the present disclosure to present means for minimizing the delay in delivery of oxygen-enriched gas to a mechanically ventilated patient, e.g. upon activation of an oxygen boost function of the ventilator. These and other objects are achieved in accordance with the present invention by a ventilator and a computer program for controlling a ventilator, as defined by the appended claims.

According to an aspect of the present disclosure there is provided a ventilator for delivering a flow of oxygen-containing breathing gas to a patient via a ventilator breathing system (VBS) connecting the ventilator and the patient. The ventilator comprises a control computer configured to control the flow of breathing gas through the VBS, and to control an oxygen concentration of the breathing gas based on a set oxygen concentration. In order to minimize the delay in delivery of a changed oxygen concentration to the patient, the control computer is configured to automatically increase the flow of breathing gas through the VBS in response to a change in the set oxygen concentration from a first oxygen concentration to a second (new) oxygen concentration.

By configuring the ventilator to automatically increase the flow of breathing gas through the VBS (hereinafter referred to as the VBS flow) in response to a change in the set oxygen concentration, the time of delivery of the new gas composition (i.e. breathing gas having the new oxygen concentration) to the patient can be efficiently reduced.

The VBS flow may be automatically increased in response to a change in set oxygen concentration no matter whether the oxygen concentration is manually changed by an operator of the ventilator or automatically changed by the control computer of the ventilator, e.g. in response to measurements obtained by one or more sensors, or in response to activation of an automatic procedure or manoeuvre performed by the ventilator.

The control computer may be configured to temporarily increase the VBS flow in response to the change in set oxygen concentration, from a set flow to an increased flushing flow. The control computer may further be configured to automatically return to the set flow when a flush deactivation condition is met, e.g. a flush deactivation condition indicating that breathing gas having the new oxygen concentration reaches or soon will reach the patient. An effect of these features is a fully automatic procedure according to which the patient receives the new oxygen concentration with a minimum of delay while minimizing breathing gas consumption and preventing improper flow settings after the procedure.

The control computer may, for instance, be configured to return to the set flow upon reception of a signal from a sensor indicating that breathing gas having the new oxygen concentration reaches or soon will reach the patient. In one example, the sensor is a gas composition sensor for measuring the oxygen concentration or a change in oxygen concentration in inspiration gas delivered towards the patient, e.g. a gas composition sensor disposed in an inspiratory line or a patient end adaptor of the VBS

Alternatively, the control computer may be configured to return to the set flow after a predetermined period of time from the change in set oxygen concentration, or after a predetermined number of breaths following the change in set oxygen concentration. For example, the control computer may be configured to return to the set flow after a predetermined time period of 15 seconds, or after five breaths. The predetermined period of time or number of breaths may be automatically determined by the control computer based on a volume of the VBS and the increased VBS flow.

Preferably, the flushing procedure is a volume-neutral flushing procedure in the meaning that the volume of breathing gas delivered to the patient is not affected by the increased flow. To this end, the control computer may be configured to regulate an inspiratory valve and/or an expiratory valve of the ventilator to increase the VBS flow in response to the change in set oxygen concentration without substantially changing a tidal volume or a minute volume of breathing gas delivered to the patient.

In one example, this is achieved by the control computer by increasing the VBS flow during one or more expiratory phases of respiration. In this way, during expiration, breathing gas having the first oxygen concentration and occupying an inspiratory line of the VBS will be effectively pushed out of the inspiratory line and into an expiratory line of the VBS by an increased bias flow of breathing gas having the new oxygen concentration, thereby minimizing the delay in delivery of breathing gas having the second and new oxygen concentration to the patient.

Instead or in addition to increasing a bias flow during expiration, the control computer is configured to increase a flow of breathing gas through the VBS during one or more inspiratory phases of respiration while controlling an expiratory valve of the ventilator in a pressure limited approach to reach a desired airway pressure during each of the one or more inspiratory phases.

To ensure efficient flushing of breathing gas having a previously set oxygen concentration out of the VBS, the flow of breathing gas through the VBS should be increased rather significantly in response to the change in set oxygen concentration. To this end, the control computer may be configured to increase the flow of breathing gas through the VBS by a factor of at least two, preferably by a factor of at least five, and most preferably by a factor of 5-20, in response to the change in set oxygen concentration.

The control computer may further be configured to determine the magnitude of the change in oxygen concentration from the first oxygen concentration to the new oxygen concentration, and to increase the flow of breathing gas through the VBS only in response to a change in oxygen concentration exceeding a predetermined threshold value. The control computer may also be configured to determine whether the change in set oxygen concentration is an increase or a decrease in set oxygen concentration, and to increase the flow of breathing gas through the VBS only in response to an increase in set oxygen concentration. However, it may be important to minimize the time of delivery of breathing gas having a changed oxygen concentration also in situations where the oxygen concentration has been decreased. For instance, it may be important to minimize the time of delivery of breathing gas having a decreased oxygen concentration to neonates exhibiting symptoms of hyperoxia.

The proposed flushing procedure is particularly intended to minimize the time of delivery of oxygen-enriched gas to the patient in situations where the patient is deemed to suffer from severe oxygen deprivation. In such situations, the oxygen concentration of the breathing gas is normally significantly increased to avoid hypoxia, e.g. through manual or automatic activation of an oxygen boost function of the ventilator, as described above. Increasing the flow of breathing gas through the VBS only in response to relatively large changes in set oxygen concentration and/or only in response to increases in set oxygen concentration has the effect of minimizing the time of delivery of oxygen-enriched gas to the patient in situations where the change in set oxygen concentration indicates that the patient may be suffering from severe oxygen deprivation, while avoiding a flushing procedure and thus alteration of ventilator settings and operation in situations where the potential delay in delivery of a new oxygen concentration gas does not jeopardize patient safety. The proposed flushing procedure may thus be advantageously used to minimize a delay of delivery of oxygen-enriched gas to the ventilated patient upon activation of an oxygen boost function of the ventilator. In response to activation of the oxygen boost function, the control computer may be configured to automatically change the set oxygen concentration of the breathing gas from a current oxygen concentration (e.g. 21% when using air as breathing gas) to an increased oxygen concentration typically corresponding to a preset oxygen boost level, such as 100% or nearly 100% oxygen. Furthermore, in response to the change in set oxygen concentration, the control computer automatically increases the flow of breathing gas (having the increased oxygen concentration) through the VBS in accordance with the principles of the present disclosure. In this way, the oxygen-enriched breathing gas can be delivered to the patient with a minimum of delay.

According to another unclaimed aspect of the present disclosure there is provided a method for controlling a ventilator delivering oxygen-containing breathing gas to a patient via a VBS connecting the ventilator and the patient. The method comprises the steps of delivering a flow of breathing gas having a set oxygen concentration through the VBS, registering a change in the set oxygen concentration from a first oxygen concentration to a second and new oxygen concentration, and automatically increasing the flow of breathing gas through the VBS in response to the change in set oxygen concentration.

The method may comprise temporarily increasing the flow of breathing gas through the VBS from a set flow to an increased flushing flow in response to the change in set oxygen concentration, and automatically returning to the set flow when a flush deactivation condition is met, e.g. a flush deactivation condition indicating that breathing gas having the new oxygen concentration reaches or soon will reach the patient.

The method may comprise returning to the set flow upon reception of a signal from a sensor indicating that breathing gas having the new oxygen concentration reaches or soon will reach the patient.

The method may comprise returning to the set flow after a predetermined period of time from the change in set oxygen concentration, or after a predetermined number of breaths following the change in set oxygen concentration.

The method may comprise determining the predetermined period of time or number of breaths based on a volume of the VBS and the flow of the increased flow of breathing gas.

The method may comprise regulating an inspiratory valve and/or an expiratory valve of the ventilator to increase the flow of breathing gas through the VBS without substantially changing a tidal volume or minute volume of breathing gas delivered to the patient.

The method may comprise increasing the flow of breathing gas through the VBS by a factor of at least two, preferably by a factor of at least five, and most preferably by a factor of 5-20, in response to the change in set oxygen concentration.

The method may comprise increasing the flow of breathing gas through the VBS by increasing a bias flow of breathing gas through the VBS during expiratory phases of respiration, whereas it comprises increasing a flow of breathing gas through the VBS during inspiratory phases of respiration while controlling an expiratory valve of the ventilator in a pressure limited approach to reach a desired airway pressure during the inspiratory phases.

The method may comprise determining the magnitude of the change in oxygen concentration from the first oxygen concentration to the second and new oxygen concentration, and increasing the flow of breathing gas through the VBS only in response to a change in oxygen concentration exceeding a predetermined threshold value. Alternatively or additionally, the method may comprise determining whether the change in oxygen concentration from the first oxygen concentration to the second and new oxygen concentration is an increase or a decrease in oxygen concentration, and increasing the flow of breathing gas through the VBS only in response to an increase in oxygen concentration.

As mentioned above, the proposed flushing procedure may be advantageously used to minimize a delay of delivery of oxygen-enriched breathing gas to the patient upon activation of an oxygen boost function of the ventilator.

The method is typically a computer-implemented method performed by the control computer upon execution of a computer program. The computer program may, for instance, be stored in a non-volatile memory of the ventilator. Thus, according to another aspect of the present disclosure, there is provided a computer program for controlling a ventilator delivering oxygen-containing breathing gas to a patient via a VBS connecting the breathing apparatus and the patient. The ventilator comprises a control computer configured to control a flow of the breathing gas through the VBS and to control an oxygen concentration of the breathing gas based on a set oxygen concentration. The computer program comprises instructions which, when executed by the control computer, causes the control computer to automatically increase the flow of breathing gas through the VBS in response to a change in the set oxygen concentration from a first oxygen concentration to a second and new oxygen concentration.

The computer program may further comprise instructions which, when executed by the control computer, causes the ventilator to perform any of, or any combinations of, the above mentioned method steps.

More advantageous aspects of the proposed ventilator, method and computer program will be described in the detailed description of embodiments following hereinafter.

### BRIEF DESCRIPTION OF THE DRAWINGS

The present disclosure will become more fully understood from the detailed description provided hereinafter and the accompanying drawings which are given by way of illustration only. In the different drawings, same reference numerals correspond to the same element.
Fig. 1 illustrates a ventilator for delivering a flow of oxygen-containing breathing gas to a patient via a VBS connecting the ventilator and the patient.
Fig. 2 is a flow chart illustrating a method for controlling a ventilator delivering oxygen-containing breathing gas to a patient via a VBS connecting the ventilator and the patient.

### DETAILED DESCRIPTION

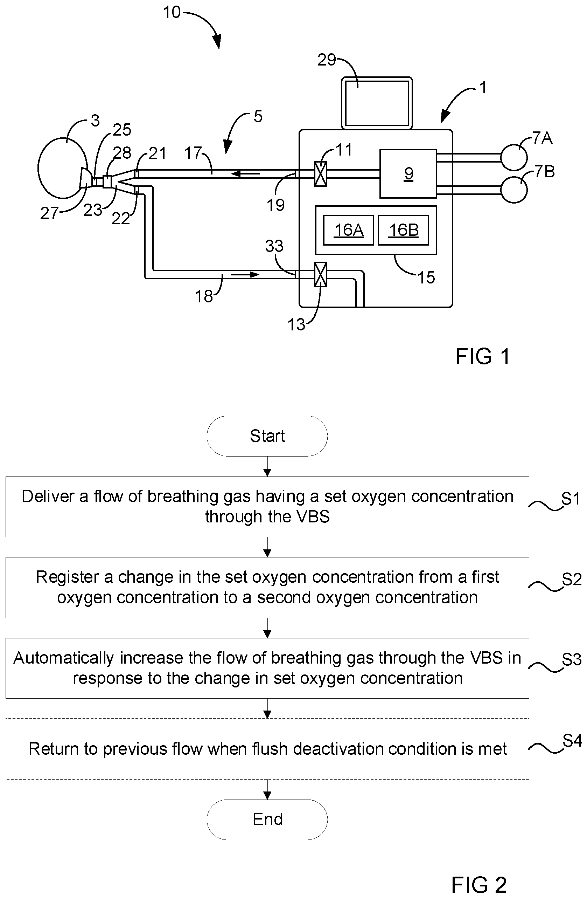

Fig. 1 illustrates a ventilation system 10 comprising a ventilator 1 connected to a patient 3 via a ventilator breathing system (VBS) 5 for delivery of breathing gas to the patient 3.

The ventilator 1 is configured for delivery of oxygen-containing breathing gas to the patient 3 via the VBS 5. In this exemplary embodiment, the ventilator 1 is connected to two gas sources 7A and 7B, such as wall outlets for the supply of pressurized gas often available in medical care facilities (medical pipeline system), compressors or gas tanks. The first gas source 7A may be an oxygen source for the supply of oxygen or oxygen-enriched breathing gas to the patient 3, and the second gas source 7B may be an air source for the supply of air or oxygen-enriched air to the patient 3.

The ventilator 1 comprises a gas mixing module 9 for regulating the composition of the breathing gas to be delivered to the patient 3. The breathing gas composition may, for instance, be air (approximately 21% oxygen), oxygen-enriched air (>21 % oxygen), or pure oxygen (100% oxygen). Of course, the ventilator 1 may be connected to additional gas sources in order to deliver other types of oxygen-containing breathing gas compositions to the ventilated patient 3.

The ventilator 1 further comprises an inspiratory valve 11 for regulating the flow and/or pressure of breathing gas delivered to the patient 3, and an expiratory valve 13 for regulating an expiratory pressure applied to the patient 3 during expiration.

Yet further, the ventilator 1 comprises a computerized control unit, herein referred to as the control computer 15, for controlling the operation of the ventilator 1, including the operation of the gas mixing module 9, the inspiratory valve 11 and the expiratory valve 13, based on ventilator settings and sensor data obtained by various sensors of the ventilation system 10.

The control computer 15 comprises at least one memory 16A, e.g. in form of a non-volatile memory hardware device, storing one or more computer programs comprising computer-readable instructions for controlling the operation of the breathing apparatus 1, and at least one processor 16B for execution of the one or more computer programs. Unless stated otherwise, actions and method steps described herein are performed by, or caused by, the control computer 15 upon execution by the processor 16B of one or more computer programs stored in the memory 16A.

The VBS 5 connecting the ventilator 1 with the patient 3 comprises an inspiratory line 17 for conveying breathing gas from the ventilator 1 towards the patient 3. The inspiratory line 17 comprises an inspiratory length of tubing connecting an inspiratory port (outlet) 19 of the ventilator 1 with an inspiratory port (inlet) 21 of a patient end adaptor 23, such as a Y-piece, as well as a portion of the patient end adaptor 23 connecting said inspiratory port 21 with a patient connection port 25 of the patient end adaptor. The patient connection port 25 of the patient end adaptor 23 is in turn connected to a patient connector 27 connecting the VBS 5 to the airways of the patient 3. The patient connector 27 may be a facemask, a tracheal tube, nasal prongs or any other type of patient connector known in the art.

The VBS 5 further comprises an expiratory line 18 for conveying expiration gas exhaled by the patient 3, as well as any bias flow of breathing gas delivered towards the patient 3 via the inspiratory line 17 during expiration phases, away from the patient 3. In this example, the expiratory line 17 comprises an expiratory length of tubing connecting an expiratory port (outlet) 22 of the patient end adaptor 23 with an expiratory port (inlet) 33 of the ventilator, as well as a portion of the patient end adaptor 23 connecting the patient connection port 25 with the expiratory port 22 of the patient end adaptor 23.

It should be appreciated that the VBS configuration illustrated in Fig. 1 is only exemplary and that the VBS 5 connecting the ventilator 1 and the patient 3 may be configured in many different ways. In all circumstances, the VBS 5 comprises an inspiratory line for conveying breathing gas from the ventilator to the patient, a patient connection port for connection of the VBS to the airways of the patient 3, and an exhaust port for exhalation gases.

The ventilator 1 is configured to deliver oxygen-containing breathing gas having a set oxygen concentration to the patient 3. The control computer 15 controls the gas mixing module 9 based on the set oxygen concentration such that breathing gas having the set oxygen concentration is delivered towards the patient 3 via the inspiratory line 17 of the VBS 5. The set oxygen concentration may be manually set by an operator of ventilator 1, or be automatically set by the control computer 15 based on measurements obtained by various sensors of the ventilation system 10. The oxygen concentration may be set e.g. in terms of fraction of inspired oxygen (FiO2).

In accordance with the principles of the present disclosure, the control computer 15 is configured to automatically increase the flow of breathing gas through the VBS 5 in response to a change in the set oxygen concentration. When the set oxygen concentration is changed from a first oxygen concentration to a second oxygen concentration (herein also referred to as "the new" oxygen concentration), the control computer 15 first effectuates the change in oxygen concentration, e.g. by controlling the gas mixing module 9 to generate breathing gas having the new oxygen concentration, and then increases the flow of breathing gas having the new oxygen concentration through the VBS 5.

This has the effect of efficiently pushing breathing gas having the previously set oxygen concentration out of the inspiratory line 17 of the VBS while filling the inspiratory line 17 with breathing gas having the new oxygen concentration, thereby minimizing the number of breaths required for breathing gas having the new oxygen concentration to reach the patient, especially if the volume of the inspiratory line 17 of the VBS 5 is large compared to the tidal volume of the patient 3.

The flow of breathing gas through the VBS 5 may hereinafter sometimes be referred to as the VBS flow, and an increased VBS flow that is delivered through the VBS for flushing breathing gas having a previously set oxygen concentration out of the inspiratory line 17 of the VBS may hereinafter sometimes be referred to as a VBS flushing flow. The procedure of delivering a VBS flushing through the VBS 5 may hereinafter sometimes be referred to as a flushing procedure or VBS flushing procedure. The control computer 15 is thus configured to automatically perform a flushing procedure in response to a change in the set oxygen concentration of the breathing gas.

The control computer 15 is preferably configured to perform a flushing procedure only in situations when a delay in delivery of breathing gas having the new oxygen concentration may jeopardize patient safety. To this end, the control computer 15 may be configured to perform a flushing procedure in response to a change in oxygen concentration only if one or more flushing criteria are fulfilled. One flushing criterion may relate to the magnitude of change of the set oxygen concentration. Another flushing criterion may relate to the type of change (i.e. increase or decrease) made to the set oxygen concentration. For instance, the control computer 15 may be configured to perform a flushing procedure only if the magnitude of change of the set oxygen concentration exceeds a predetermined threshold value, and/or only if the change in set oxygen concentration is an increase in set oxygen concentration. In one example, the control computer 15 is configured to perform a flushing procedure only if the change in set oxygen concentration is an increase in oxygen concentration, and if the magnitude of change exceeds a predetermined threshold value, for example 5 vol%.

The flushing criterion may also relate to a calculated delay in delivery of breathing gas having the new (i.e. changed) oxygen concentration. The control computer 15 may, for instance, be configured to calculate a delay in delivery of breathing gas having the new oxygen concentration based on a volume of the inspiratory line 17 of the VBS 5 and a current flow rate. If the calculated delay exceeds a predetermined threshold value, the control computer 15 may perform a flushing procedure to minimize the delay. The volume of the inspiratory line 17 of the VBS 5 may be calculated by the control computer 15 in accordance with the principles disclosed in WO 2016/209129 A1 by the same applicant.

The VBS flushing flow should preferably be significantly higher than a patient flow used prior to the change in set oxygen concentration in order to achieve the desired effect of efficiently flushing breathing gas having the previous (old) oxygen concentration out of the inspiratory line 17 of the VBS. For instance, the control computer 15 may be configured to set the VBS flushing flow to at least five times the patient flow used prior to the change in oxygen concentration. Preferably, the VBS flushing flow is set to a flow of 5-20 times the patient flow used prior to the change in oxygen concentration. The VBS flushing flow is thus significantly higher than the flow required for adequate ventilation of the patient 3.

The flushing procedure is preferably a volume-neutral procedure in the meaning of not substantially affecting the volume of breathing gas delivered to the patient. This means that the patient 3 receives substantially the same volume of breathing gas, e.g. in terms of both tidal volume and minute volume, during the VBS flushing procedure as during baseline ventilation provided to the patient 3 prior to the flushing procedure.

The VBS flushing flow may be an inspiratory flow that is delivered during one or more inspiratory phases, and/or a bias flow that is delivered during one or more expiratory phases.

For instance, during expiratory phases, the control computer 15 may be configured to deliver a VBS flushing flow in form of an increased bias flow, hereinafter referred to as a VBS bias flushing flow. The VBS bias flushing flow may be 5-20 times a bias flow used during baseline ventilation of the patient 3 prior to the flushing procedure. For example, if a bias flow of 0.5 L/min is used during baseline ventilation of the patient, the control computer 15 may be configured to increase the bias flow so as to deliver a VBS bias flushing flow of 2.5-10 L/min through the VBS 5 in response to a change in the set oxygen concentration.

Instead or in addition to a VBS bias flushing flow that is delivered during expiratory phases, the control computer 15 is configured to deliver a VBS flushing flow in form of an increased inspiratory flow during inspiratory phases, hereinafter referred to as a VBS inspiratory flushing flow. In this scenario, in order maintain desired tidal volume and minute ventilation of the patient 3, the control computer is configured to control the expiratory valve 13 of the ventilator 1 in a pressure limited approach where the expiratory valve 13 is regulated to limit the inspiratory pressure. This means that the expiratory valve 13 is regulated during the expiratory phase so as to maintain a desired airway pressure of the patient 3 during delivery of the VBS inspiratory flushing flow, e.g. to maintain a set airway pressure used during pressure-controlled baseline ventilation prior to the flushing procedure. In this scenario, parts of the VBS inspiratory flushing flow will be inhaled by the patient 3, whereas parts (the main part) of the VBS inspiratory flushing flow will pass through the patient end adaptor 23 and into the expiratory line 18 without reaching the patient. The VBS inspiratory flushing flow may be 5-20 times an inspiratory flow used during baseline ventilation of the patient 3 prior to the flushing procedure. For example, if an inspiratory flow of 6 L/min is used during baseline ventilation of the patient, the control computer 15 may be configured to increase the inspiratory flow so as to deliver a VBS inspiratory flushing flow of 60 L/min through the VBS 5 in response to a change in the set oxygen concentration.

If the ventilator 1 is operated in a volume controlled mode or in another operational mode in which flushing of the VBS is deemed inappropriate or cannot easily be performed without substantially affecting the ongoing ventilatory treatment of the patient 3, the control computer 15 may, in response to the change in set oxygen concentration, be configured to switch operational mode of the ventilator 1 from a current operational mode to a pressure limited mode, before initiating the VBS flushing procedure. In this way, the control computer 15 can control the expiratory valve 13 in accordance with the above mentioned pressure limited approach to ensure that the level of ventilation of the patient 3 is not affected to any substantial extent by the VBS flushing procedure. The proposed VBS flushing procedure may thus comprise a step of automatically switching from a current operational mode to a pressure limited operational mode before increasing the flow of breathing gas through the VBS.

The duration of the flushing procedure may be predetermined. For example, the control computer 15 may be configured to deliver a VBS flushing flow (e.g. a VBS bias flushing flow and/or a VBS inspiratory flushing flow) during a predetermined period of time after the change in set oxygen concentration, or during a predetermined number of breaths following the change in set oxygen concentration. In one example, the control computer 15 is configured to deliver a VBS bias flushing flow during 3-7 and preferably during about five expiratory phases following the change in set oxygen concentration, and/or to deliver a VBS inspiratory flushing flow during 2-6 and preferably about four inspiratory phases following the change in set oxygen concentration.

The control computer 15 may further be configured to determine the duration of the flushing procedure based on the flow rate of the VBS flow and the volume of at least the inspiratory line 17 of the VBS 5. Based on these parameters, the control computer 15 can calculate a point in time when breathing gas having the new oxygen concentration is expected to reach the patient 3, and to interrupt the flushing procedure at or near that point in time. The volume of the inspiratory line 17 of the VBS 5 may be determined by the control computer 15 in any way known in the art. For example, the control computer 15 may determine the volume of the inspiratory line 17 based on user input received from an operator of the ventilator 1, or to determine the volume automatically based on a pressure/volume relationship of the VBS 5, e.g. as suggested in WO 2016/209129 A1.

Alternatively, the duration of the flushing procedure is determined based on real-time or near real-time sensor data obtained by one or more sensors of the ventilation system 10 and indicative of the oxygen concentration or a rate of change in oxygen concentration of the breathing gas that reaches or soon will reach the patient 3.

The control computer 15 may be configured to interrupt the flushing procedure upon reception of a signal from a sensor indicating that breathing gas having the new oxygen concentration reaches or soon will reach the patient 3.

For example, the control computer 15 may be configured to determine when to interrupt the flushing procedure based on sensor data obtained by a gas composition sensor 28 disposed in the inspiratory line 17 of the VBS 5. In the illustrated example, the gas composition sensor 28 is a proximal sensor, meaning that it is arranged in or near the patient end adaptor 23. Using a proximal gas composition sensor is advantageous in that the sensor data becomes indicative of the oxygen concentration of breathing gas that is to be delivered to the patient 3 very shortly.

Alternatively, the gas composition sensor 28 may be disposed in the expiratory line 18 of the VBS 5, and the control computer 15 may be configured to determine when to interrupt the flushing procedure based on sensor data obtained by the gas composition sensor 28 during inspiration in the above mentioned pressure limited mode of operation of the ventilator 1. In this case, the gas measured upon will be breathing gas flowing directly into the expiratory line 18 from the inspiratory line 17, without reaching the airways of the patient 3. Consequently, in this scenario, the oxygen concentration of the gas measured upon will correspond to the oxygen concentration of the breathing gas that reaches or already has reached the patient 3, thereby allowing the control computer 15 to interrupt the flushing procedure when it can be asserted that the oxygen concentration of the breathing gas reaching the patient 3 has reached or exceeds a predetermined threshold value.

As a non-limiting example, the gas composition sensor 28 may be an ultrasound sensor for measuring the time of flight (TOF) of an ultrasound pulse propagating through the breathing gas. As the oxygen concentration in the breathing gas changes due to the change in set oxygen concentration, the TOF measured by the gas composition sensor 28 will change accordingly. When the oxygen concentration in the breathing gas approaches the new set oxygen concentration, the rate of change in oxygen concentration decreases, causing the rate of change in measured TOF to decrease accordingly. The control unit 15 may therefore, in one example, be configured to interrupt the flushing procedure when the rate of change of the measured TOF falls below a preset threshold value.

The flushing procedure is typically interrupted by the control computer 15 by removing the increase in flow through the VBS 5 that was made in response to the change in set oxygen concentration. Thereby, upon interruption of the flushing procedure, the flow of breathing gas through the VBS 5 will return to the set flow used during baseline ventilation prior to the flushing procedure. Consequently, upon interruption of the flushing procedure, any VBS bias flushing flow may be removed and the bias flow may be set to correspond to the bias flow used prior to the flushing procedure, and any VBS inspiratory flushing flow may be removed and the inspiratory flow (patient flow) may be set to correspond to the inspiratory flow used prior to the flushing procedure.

The proposed flushing procedure is particularly intended to be used as part of an oxygen boost procedure, which may be initiated in case the ventilated patient 3 is in need of a temporary increase in oxygen supply (i.e. an "oxygen boost").

The control computer 15 may be configured to carry out an oxygen boost procedure by temporarily increasing the oxygen concentration of the breathing gas delivered to the patient 3 upon activation of an oxygen boost function of the ventilator 1. When the oxygen boost function is activated, the control computer 15 may be configured to control the ventilator 1 to temporarily increase the oxygen concentration of the breathing gas from a baseline level used prior to activation of the oxygen boost function to a desired and increased level of oxygen concentration (the oxygen boost level), and, when one or more oxygen boost abortion criteria are met, return to the baseline level of oxygen concentration. The oxygen boost level may be predefined (e.g. 100% oxygen) or selectable by an operator of the ventilator 1.

The ventilator 1 may be configured to automatically activate the oxygen boost function upon detection of a situation in which certain criteria indicating an increased demand for oxygen by the patient 3 is met, e.g. when at least one sensor-monitored parameter indicative of increased demand for oxygen by the patient 3 exceeds a predetermined threshold value. Typically, however, the oxygen boost function is activated manually by the operator of the ventilator 1, e.g. by pressing an actuator of the ventilator. For instance, the oxygen boost function may be activated by the operator by pressing a touch-button displayed on a touch-sensitive screen of a ventilator display unit 29.

When the oxygen boost function is activated, the control computer 15 changes the set oxygen concentration to correspond to the oxygen boost level. The change in set oxygen concentration triggers the control computer 15 to perform a flushing procedure as outlined above, thereby minimizing the delay of delivery of the oxygen boost to the patient.

Fig. 2 is a flowchart illustrating an unclaimed e- method for controlling a ventilator delivering oxygen-containing breathing gas to a patient via a VBS connecting the ventilator and the patient. The method is not limited to any specific ventilation system setup but will, by way of example, be described in the context of the ventilation system 10 shown in Fig. 1.

The method is typically a computer-implemented method that is performed by the control computer 15 of the ventilator 1 upon execution by the processor 16B of a computer program stored in the memory 16A.

Furthermore, as discussed above, the method may or may not form part of a method for providing improved oxygen boost functionality of a ventilator delivering oxygen-containing breathing gas to a patient via a VBS connecting the ventilator and the patient. Such a method for improved oxygen boost functionality may involve performing the method steps described hereinafter in response to activation of an oxygen boost function of the ventilator.

In a first step, S1, a flow of breathing gas having a set oxygen concentration is delivered through the VBS 5 towards the patient 3. The flow of breathing gas is delivered towards the patient 3 via the inspiratory line 17 of the VBS 5. The flow is typically adapted to the patient 3 and the ongoing ventilatory treatment and is normally referred to as either of an inspiratory flow or a patient flow. Step S1 is performed during a period of baseline ventilation of the patient 3.

In a second step, S2, a change in the set oxygen concentration is registered. As discussed above, the change may be manually performed by an operator of the ventilator 1, or automatically performed by the control computer 15 itself in response to sensor measurements obtained by one or more sensors of the ventilation system 10 or in response to activation of an automatic procedure or manoeuvre requiring the oxygen concentration to be changed, such as an oxygen boost procedure.

In a third step, S3, the flow of breathing gas through the VBS 5 is automatically increased in response to the change in set oxygen concentration registered in step S2. That the flow through the VBS 5 is increased means that at least the flow of breathing gas through the inspiratory line 17 of the VBS 5 is increased. Typically, in the event of a volume-neutral flushing procedure as discussed above, this means that the flow through the expiratory line 18 of the VBS is increased to substantially the same extent. As also discussed above, the increase in flow may involve an increase in any or both of an inspiratory flow of breathing gas delivered through the VBS 5 during inspiratory phases, and a bias flow delivered through the VBS 5 during expiratory phases.

The increased flow of breathing gas delivered through the VBS in step S3 is a flow of breathing gas having the new oxygen concentration. To this end, the method may comprise an optional step (not shown), preceding step S3, of effectuating the change in set oxygen concentration registered in step S2, i.e. a step of actually changing the set oxygen concentration from the first oxygen concentration to the second and new oxygen concentration. This may be achieved by the control computer 15 by controlling the gas mixing module 9 to produce breathing gas having the new oxygen concentration.

The method may further comprise a subsequent and final step, S4, of automatically returning to the patient flow used during baseline ventilation when a flush deactivation condition is met, e.g. a flush deactivation condition indicating that breathing gas having the new oxygen concentration reaches or soon will reach the patient.

## Claims

1. A ventilator (1) for delivering a flow of oxygen-containing breathing gas to a patient (3) via a ventilator breathing system (5), VBS, connecting the ventilator and the patient, the ventilator comprising a control computer (15) configured to control the flow of breathing gas through the VBS and to control an oxygen concentration of the breathing gas based on a set oxygen concentration, wherein the control computer (15) is configured to automatically increase the flow of breathing gas through the VBS in response to a change in the set oxygen concentration from a first oxygen concentration to a second oxygen concentration, **characterized by** increasing a flow of breathing gas through the VBS during one or more inspiratory phases of respiration while controlling an expiratory valve (13) of the ventilator in a pressure limited approach to reach a desired airway pressure during each of the one or more inspiratory phases.

2. The ventilator (1) of claim 1, wherein the control computer (15) is configured to temporarily increase the flow of breathing gas through the VBS (5) from a set flow to an increased flushing flow in response to the change in set oxygen concentration, and to automatically return to the set flow when a flush deactivation condition is met.

3. The ventilator (1) of claim 2, wherein the control computer (15) is configured to return to the set flow upon reception of a signal from a sensor (27) indicating that breathing gas having the new oxygen concentration reaches or soon will reach the patient (3).

4. The ventilator (1) of claim 2, wherein the control computer (15) is configured to return to the set flow after a predetermined period of time from the change in set oxygen concentration, or after a predetermined number of breaths following the change in set oxygen concentration.

5. The ventilator (1) of claim 4, wherein the control computer (15) is configured to determine the period of time or the number of breaths based on a volume of the VBS (5) and a flow rate of the increased flow of breathing gas.

6. The ventilator (1) of any of the preceding claims, wherein the control computer (15) is configured to regulate an inspiratory valve (11) and/or an expiratory valve (13) of the ventilator to increase the flow of breathing gas through the VBS (15) without substantially changing a tidal volume or minute volume of breathing gas delivered to the patient (3).

7. The ventilator (1) of any of the preceding claims, wherein the control computer (15) is configured to increase the flow of breathing gas through the VBS (5) by increasing a bias flow of breathing gas through the VBS during one or more expiratory phases of respiration.

8. The ventilator (1) of any of the preceding claims, wherein the control computer (15) is configured to increase the flow of breathing gas through the VBS by a factor of at least two, and preferably by a factor of at least five, in response to the change in set oxygen concentration.

9. The ventilator (1) of any of the preceding claims, wherein the control computer (15) is configured to determine the magnitude of the change in oxygen concentration from the first oxygen concentration to the second and new oxygen concentration, and to increase the flow of breathing gas through the VBS (5) only in response to a change in oxygen concentration exceeding a predetermined threshold value.

10. A computer program for controlling a ventilator (1) delivering oxygen-containing breathing gas to a patient (3) via a ventilator breathing system (5), VBS, connecting the ventilator and the patient, the computer program comprising computer-readable instructions which, when executed by a control computer (15) of the ventilator, causes the ventilator to perform the steps of:
- delivering a flow of breathing gas having a set oxygen concentration through the VBS;
- registering a change in the set oxygen concentration from a first oxygen concentration to a second oxygen concentration,
- automatically increasing the flow of breathing gas through the VBS in response to the change in set oxygen concentration, **characterized by** increasing a flow of breathing gas through the VBS during one or more inspiratory phases of respiration while controlling an expiratory valve (13) of the ventilator in a pressure limited approach to reach a desired airway pressure during each of the one or more inspiratory phases.

11. The computer program of claim 10, which, when executed by the control computer (15), causes the ventilator to perform the steps of:
- temporarily increasing the flow of breathing gas through the VBS (5) from a set flow to an increased flushing flow in response to the change in set oxygen concentration, and
- automatically returning to the set flow when a flush deactivation condition is met.

12. The computer program of claim 11, which, when executed by the control computer (15), causes the ventilator to return to the set flow upon reception of a signal from a sensor (27) indicating that breathing gas having the new oxygen concentration reaches or soon will reach the patient (3).

13. The computer program of claim 11, which, when executed by the control computer (15), causes the ventilator to return to the set flow after a predetermined period of time from the change in set oxygen concentration, or after a predetermined number of breaths following the change in set oxygen concentration.

14. The computer program of claim 13, which, when executed by the control computer (15), causes the ventilator to determine the predetermined period of time or number of breaths based on a volume of the VBS (15) and the flow of the increased flow of breathing gas.

15. The computer program of any of the claims 10-14, which, when executed by the control computer (15), causes the ventilator to regulate an inspiratory valve (11) and/or an expiratory valve (13) of the ventilator (1) to increase the flow of breathing gas through the VBS (15) without substantially changing a tidal volume or minute volume of breathing gas delivered to the patient (3).

## Patentansprüche

1. Beatmungsgerät (1) zum Zuführen eines Flusses von Sauerstoff enthaltendem Atemgas an einen Patienten (3) über ein Beatmungssystem (5), VBS, das das Beatmungsgerät und den Patienten verbindet, wobei das Beatmungsgerät einen Steuerungscomputer (15), der dazu konfiguriert ist, den Fluss von Atemgas durch das VBS zu steuern und eine Sauerstoffkonzentration des Atemgases basierend auf einer eingestellten Sauerstoffkonzentration zu steuern, umfasst, wobei der Steuerungscomputer (15) dazu konfiguriert ist, den Fluss von Atemgas durch das VBS in Reaktion auf eine Änderung der eingestellten Sauerstoffkonzentration von einer ersten Sauerstoffkonzentration zu einer zweiten Sauerstoffkonzentration automatisch zu erhöhen, **gekennzeichnet durch** Erhöhen eines Flusses von Atemgas durch das VBS während einer oder mehrerer Einatmungsphasen der Atmung, bei gleichzeitigem Steuern eines Ausatemventils (13) des Beatmungsgeräts in einem druckbegrenzten Ansatz zum Erreichen eines gewünschten Atemwegsdrucks während jeder der einen oder mehreren Einatmungsphasen.

2. Beatmungsgerät (1) nach Anspruch 1, wobei der Steuerungscomputer (15) dazu konfiguriert ist, den Fluss von Atemgas durch das VBS (5) zeitweise von einem eingestellten Fluss zu einem erhöhten Flush-Fluss in Reaktion auf die Änderung der eingestellten Sauerstoffkonzentration zu erhöhen und automatisch zu dem eingestellten Fluss zurückzukehren, wenn eine Flush-Deaktivierungsbedingung erfüllt ist.

3. Beatmungsgerät (1) nach Anspruch 2, wobei der Steuerungscomputer (15) dazu konfiguriert ist, bei Empfang eines Signals von einem Sensor (27), das angibt, dass Atemgas, das die neue Sauerstoffkonzentration aufweist, den Patienten (3) erreicht oder bald erreichen wird, zu dem eingestellten Fluss zurückzukehren.

4. Beatmungsgerät (1) nach Anspruch 2, wobei der Steuerungscomputer (15) dazu konfiguriert ist, nach einer vorbestimmten Zeitdauer ab der Änderung der eingestellten Sauerstoffkonzentration oder nach einer vorbestimmten Anzahl von Atemzügen nach der Änderung der eingestellten Sauerstoffkonzentration zu dem eingestellten Fluss zurückzukehren.

5. Beatmungsgerät (1) nach Anspruch 4, wobei der Steuerungscomputer (15) dazu konfiguriert ist, die Zeitdauer oder die Anzahl von Atemzügen basierend auf einem Volumen des VBS (5) und einer Flussrate des erhöhten Flusses von Atemgas zu bestimmen.

6. Beatmungsgerät (1) nach einem der vorhergehenden Ansprüche, wobei der Steuerungscomputer (15) dazu konfiguriert ist, ein Einatmungsventil (11) und/oder ein Ausatmungsventil (13) des Beatmungsgeräts zu regulieren, um den Fluss von Atemgas durch das VBS (5) zu erhöhen, ohne ein Atemzugsvolumen oder Minutenvolumen von Atemgas, das dem Patienten (3) zugeführt wird, wesentlich zu ändern.

7. Beatmungsgerät (1) nach einem der vorhergehenden Ansprüche, wobei der Steuerungscomputer (15) dazu konfiguriert ist, den Fluss von Beatmungsgas durch das VBS (5) zu erhöhen, indem ein Bias-Fluss von Atemgas durch das VBS während einer oder mehrerer Ausatmungsphasen der Atmung erhöht wird.

8. Beatmungsgerät (1) nach einem der vorhergehenden Ansprüche, wobei der Steuerungscomputer (15) dazu konfiguriert ist, den Fluss von Beatmungsgas durch das VBS um einen Faktor von mindestens zwei, und bevorzugt um einen Faktor von mindestens fünf, in Reaktion auf die Änderung der eingestellten Sauerstoffkonzentration zu erhöhen.

9. Beatmungsgerät (1) nach einem der vorhergehenden Ansprüche, wobei der Steuerungscomputer (15) dazu konfiguriert ist, die Größenordnung der Änderung der Sauerstoffkonzentration von der ersten Sauerstoffkonzentration zu der zweiten und neuen Sauerstoffkonzentration zu bestimmen und den Fluss von Atemgas durch das VBS (5) nur in Reaktion auf eine Änderung der Sauerstoffkonzentration, die einen vorbestimmten Schwellenwert überschreitet, zu erhöhen.

10. Computerprogramm zum Steuern eines Beatmungsgeräts (1) zum Zuführen von Sauerstoff enthaltendem Atemgas an einen Patienten (3) über ein Beatmungssystem (5), VBS, das das Beatmungsgerät und den Patienten verbindet, wobei das Computerprogramm computerlesbare Anweisungen umfasst, die, wenn sie von einem Steuerungscomputer (15) des Beatmungsgeräts ausgeführt werden, das Beatmungsgerät dazu veranlassen, die folgenden Schritte auszuführen:
- Zuführen eines Flusses von Atemgas, das eine eingestellte Sauerstoffkonzentration aufweist, durch das VBS;
- Erfassen einer Änderung der eingestellten Sauerstoffkonzentration von einer ersten Sauerstoffkonzentration zu einer zweiten Sauerstoffkonzentration,
- automatisches Erhöhen des Flusses von Atemgas durch das VBS in Reaktion auf die Änderung der eingestellten Sauerstoffkonzentration, **gekennzeichnet durch** Erhöhen eines Flusses von Atemgas durch das VBS während einer oder mehrerer Einatmungsphasen der Atmung, bei gleichzeitigem Steuern eines Ausatemventils (13) des Beatmungsgeräts in einem druckbegrenzten Ansatz zum Erreichen eines gewünschten Atemwegsdrucks während jeder der einen oder mehreren Einatmungsphasen.

11. Computerprogramm nach Anspruch 10, das, wenn es durch den Steuerungscomputer (15) ausgeführt wird, das Beatmungsgerät dazu veranlasst, die folgenden Schritte durchzuführen:
- zeitweises Erhöhen des Flusses von Atemgas durch das VBS (5) von einem eingestellten Fluss zu einem erhöhten Flush-Fluss in Reaktion auf die Änderung der eingestellten Sauerstoffkonzentration, und
- automatisches Zurückkehren zu dem eingestellten Fluss, wenn eine Flush-Deaktivierungsbedingung erfüllt ist.

12. Computerprogramm nach Anspruch 11, das, wenn es durch den Steuerungscomputer (15) ausgeführt wird, das Beatmungsgerät dazu veranlasst, bei Empfang eines Signals von einem Sensor (27), das angibt, dass Atemgas, das die neue Sauerstoffkonzentration aufweist, den Patienten (3) erreicht oder bald erreichen wird, zu dem eingestellten Fluss zurückzukehren.

13. Computerprogramm nach Anspruch 11, das, wenn es durch den Steuerungscomputer (15) ausgeführt wird, das Beatmungsgerät dazu veranlasst, nach einer vorbestimmten Zeitdauer ab der Änderung der eingestellten Sauerstoffkonzentration oder nach einer vorbestimmten Anzahl von Atemzügen nach der Änderung der eingestellten Sauerstoffkonzentration zu dem eingestellten Fluss zurückzukehren.

14. Computerprogramm nach Anspruch 13, das, wenn es durch den Steuerungscomputer (15) ausgeführt wird, das Beatmungsgerät dazu veranlasst, die vorbestimmte Zeitdauer oder Anzahl von Atemzügen basierend auf einem Volumen des VBS (5) und einer Flussrate des erhöhten Flusses von Atemgas zu bestimmen.

15. Computerprogramm nach einem der Ansprüche 10-14, das, wenn es durch den Steuerungscomputer (15) ausgeführt wird, das Beatmungsgerät dazu veranlasst, ein Einatmungsventil (11) und/oder ein Ausatmungsventil (13) des Beatmungsgeräts (1) zu regulieren, um den Fluss von Atemgas durch das VBS (5) zu erhöhen, ohne ein Atemzugsvolumen oder ein Minutenvolumen von Atemgas, das dem Patienten (3) zugeführt wird, wesentlich zu ändern.

## Revendications

1. Ventilateur (1) pour fournir un débit de gaz respiratoire contenant de l'oxygène à un patient (3) via un système respiratoire à ventilateur (5), VBS, reliant le ventilateur et le patient, le ventilateur comprenant un ordinateur de commande (15) configuré pour commander le débit de gaz respiratoire à travers le VBS et pour commander une concentration en oxygène du gaz respiratoire sur la base d'une concentration d'oxygène définie, dans lequel l'ordinateur de commande (15) est configuré pour augmenter automatiquement le débit de gaz respiratoire à travers le VBS en réponse à un changement de la concentration d'oxygène définie d'une première concentration d'oxygène à une seconde concentration d'oxygène, **caractérisé par** l'augmentation d'un débit de gaz respiratoire à travers le VBS pendant une ou plusieurs phases inspiratoires de respiration tout en commandant une valve expiratoire (13) du ventilateur dans une approche à pression limitée pour atteindre une pression des voies respiratoires souhaitée pendant chacune de l'une ou plusieurs phases inspiratoires.

2. Ventilateur (1) selon la revendication 1, dans lequel l'ordinateur de commande (15) est configuré pour augmenter temporairement le débit de gaz respiratoire à travers le VBS (5) d'un débit défini à un débit de rinçage accru en réponse au changement de la concentration d'oxygène défini, et de revenir automatiquement au débit défini lorsqu'une condition de désactivation de rinçage est remplie.

3. Ventilateur (1) selon la revendication 2, dans lequel l'ordinateur de commande (15) est configuré pour revenir au débit défini lors de la réception d'un signal d'un capteur (27) indiquant que le gaz respiratoire ayant la nouvelle concentration d'oxygène atteint ou atteindra bientôt le patient (3) .

4. Ventilateur (1) selon la revendication 2, dans lequel l'ordinateur de commande (15) est configuré pour revenir au débit défini après une durée prédéterminée à partir du changement de concentration d'oxygène définie, ou après un nombre prédéterminé de respirations à la suite du changement de concentration d'oxygène définie.

5. Ventilateur (1) selon la revendication 4, dans lequel l'ordinateur de commande (15) est configuré pour déterminer la durée ou le nombre de respirations sur la base d'un volume du VBS (5) et d'un débit de flux accru de gaz respiratoire.

6. Ventilateur (1) selon l'une quelconque des revendications précédentes, dans lequel l'ordinateur de commande (15) est configuré pour réguler une valve inspiratoire (11) et/ou une valve expiratoire (13) du ventilateur pour augmenter le débit de gaz respiratoire à travers le VBS (15) sans modifier sensiblement un volume courant ou un volume minute de gaz respiratoire fourni au patient (3).

7. Ventilateur (1) selon l'une quelconque des revendications précédentes, dans lequel l'ordinateur de commande (15) est configuré pour augmenter le débit de gaz respiratoire à travers le VBS (5) en augmentant un débit de polarisation de gaz respiratoire à travers le VBS pendant une ou plusieurs phases expiratoires de respiration.

8. Ventilateur (1) selon l'une quelconque des revendications précédentes, dans lequel l'ordinateur de commande (15) est configuré pour augmenter le débit de gaz respiratoire à travers le VBS d'un facteur d'au moins deux, et de préférence d'un facteur d'au moins cinq, en réponse au changement de concentration d'oxygène définie.

9. Ventilateur (1) selon l'une quelconque des revendications précédentes, dans lequel l'ordinateur de commande (15) est configuré pour déterminer l'ampleur du changement de concentration d'oxygène de la première concentration en oxygène à la seconde et nouvelle concentration en oxygène, et pour augmenter le débit de gaz respiratoire à travers le VBS (5) uniquement en réponse à un changement de concentration en oxygène dépassant une valeur seuil prédéterminée.

10. Programme informatique pour commander un ventilateur (1) fournissant un gaz respiratoire contenant de l'oxygène à un patient (3) via un système respiratoire à ventilateur (5), VBS, reliant le ventilateur et le patient, le programme informatique comprenant des instructions lisibles par ordinateur qui, lorsqu'il est exécuté par un ordinateur de commande (15) du ventilateur, amène le ventilateur à exécuter les étapes suivantes :
- fournir un débit de gaz respiratoire ayant une concentration d'oxygène définie à travers le VBS ;
- enregistrer un changement dans la concentration d'oxygène définie d'une première concentration d'oxygène à une seconde concentration d'oxygène,
- augmenter automatiquement le débit de gaz respiratoire à travers le VBS en réponse au changement de concentration d'oxygène définie, **caractérisé par** l'augmentation d'un débit de gaz respiratoire à travers le VBS pendant une ou plusieurs phases inspiratoires de respiration tout en commandant une valve expiratoire (13) du ventilateur dans une approche à pression limitée pour atteindre une pression des voies respiratoires souhaitée pendant chacune de l'une ou plusieurs phases inspiratoires.

11. Programme informatique selon la revendication 10, qui, lorsqu'il est exécuté par l'ordinateur de commande (15), amène le ventilateur à exécuter les étapes suivantes :
- augmenter temporairement le débit de gaz respiratoire à travers le VBS (5) d'un débit défini à un débit de rinçage accru en réponse au changement de la concentration définie en oxygène, et
- revenir automatiquement au débit défini lorsqu'une condition de désactivation du rinçage est remplie.

12. Programme informatique selon la revendication 11, qui, lorsqu'il est exécuté par l'ordinateur de commande (15), amène le ventilateur à revenir au débit défini lors de la réception d'un signal d'un capteur (27) indiquant que le gaz respiratoire ayant la nouvelle concentration d'oxygène atteint ou atteindra bientôt le patient (3).

13. Programme informatique selon la revendication 11, qui, lorsqu'il est exécuté par l'ordinateur de commande (15), amène le ventilateur à revenir au débit défini après une durée prédéterminée à partir du changement de concentration d'oxygène définie, ou après un nombre prédéterminé de respirations à la suite du changement de concentration d'oxygène définie.

14. Programme informatique selon la revendication 13, qui, lorsqu'il est exécuté par l'ordinateur de commande (15), amène le ventilateur à déterminer la durée ou le nombre de respirations prédéterminés sur la base d'un volume du VBS (15) et du débit accru de gaz respiratoire.

15. Programme informatique selon l'une quelconque des revendications 10 à 14, qui, lorsqu'il est exécuté par l'ordinateur de commande (15), amène le ventilateur à réguler une valve inspiratoire (11) et/ou une valve expiratoire (13) du ventilateur (1) pour augmenter le débit de gaz respiratoire à travers le VBS (15) sans modifier sensiblement un volume courant ou un volume minute de gaz respiratoire fourni au patient (3).
